# FASCICULE DE BREVET EUROPEEN

(11) **EP 2 660 190 B1**
(45) Date de publication et mention de la délivrance du brevet: **12.11.2014**
(21) Numéro de dépôt: 13165131.7
(22) Date de dépôt: 24.04.2013
(51) Int. Cl.: B81B 7/00, G01D 11/24, A61B 5/00, H01L 21/56

(54) **Composant à élément actif encapsulé et dispositif médical implantable comportant un tel composant**
Bauteil mit eingekapseltem aktivem Element und implantierbare medizinische Vorrichtung, die ein solches Bauteil beinhaltet
Encapsulated component with active element and implantable medical device comprising said component

(30) Priorité: 03.05.2012 FR 1254079
(43) Date de publication de la demande: 06.11.2013
(73) Titulaire: Commissariat à l'Énergie Atomique et aux Énergies Alternatives, 75015 Paris (FR); Sorin CRM SAS, 92140 Clamart Cedex (FR)
(72) Inventeur: Souriau, Jean-Charles, 38120 SAINT-EGREVE (FR); Parat, Guy-Michel, 38640 CLAIX (FR); Dal Molin, Renzo, 92230 CHATILLON (FR)
(74) Mandataire: Ilgart, Jean-Christophe

(56) Documents cités:
- EP-A2- 1 903 000
- WO-A1-97/04377
- Brian Hindman: "APPLICATION NOTE 3808 What Is an iButton?", , 1 juin 2006 (2006-06-01), XP055047087, Extrait de l'Internet: URL:http://pdfserv.maximintegrated.com/en/ an/AN3808.pdf [extrait le 2012-12-07]

## Description

### DOMAINE TECHNIQUE

L'invention concerne un composant à élément actif encapsulé comportant au moins deux liaisons électriques, pouvant être immergé en particulier dans des milieux hostiles pour l'électronique, comme par exemple destiné à être implanté dans un corps, ou une partie d'un corps, humain ou animal. Le composant peut-être biocompatible ou rendu biocompatible pour le domaine des dispositifs médicaux actifs tels que définis par la directive 93/42/CEE du 14 juin 1993, et notamment celui des dispositifs médicaux implantables actifs, tels que définis par la directive 90/385/CEE du 20 juin 1990. L'invention concerne un composant et notamment un composant biocompatible, un procédé de réalisation d'un tel composant ainsi qu'un dispositif médical implantable comportant un tel composant rendu biocompatible.

L'invention est avantageusement utilisée pour la réalisation de dispositifs médicaux actifs tels que :
- des appareils de surveillance de l'activité cardiaque et de génération d'impulsions de stimulation, de resynchronisation, de défibrillation et/ou de cardioversion en cas de détection de trouble cardiaque,
- des appareils neurologiques,
- des pompes de diffusion de substances médicales,
- des implants cochléaires,
- des capteurs biologiques implantés,
- des dispositifs de mesure de pH,
- des impédances intracorporelles (mesure d'impédance transpulmonaire ou intracardiaque).

### ÉTAT DE LA TECHNIQUE ANTÉRIEURE

Un dispositif médical implantable actif comporte un ou plusieurs éléments actifs, tels que des capteurs permettant de mesurer un ou plusieurs paramètres physiologiques et/ou de réaliser le pilotage de diverses fonctions du dispositif médical. Ces capteurs peuvent être intégrés à une sonde elle-même reliée à un boîtier de générateur du dispositif. Ces capteurs sont par exemple des capteurs de pression sanguine ou d'accélération endocardiaque montés à l'extrémité distale d'une sonde endocavitaire destinée à être introduite dans le myocarde et reliée au boîtier d'un stimulateur ou défibrillateur. Plus généralement, ces éléments actifs peuvent également être des microsystèmes électromécaniques (MEMS), des nanosystèmes électromécaniques (NEMS) ou encore des circuits électroniques intégrés, par exemple de type ASIC ou FPGA, réalisant des fonctions d'amplification de signaux, de filtrage, etc.

Afin de pouvoir être implantés dans un corps humain ou animal, les éléments actifs sont enrobés dans un matériau biocompatible (notamment étanche), c'est-à-dire un matériau qui est totalement inerte à l'égard des tissus et fluides corporels environnants et qui ne se détériore pas en dépit d'un contact prolongé avec le milieu biologique environnant.

Certaines normes exigent en outre que le packaging des éléments actifs soit réalisé avec un double niveau de protection présentant une biocompatibilité non seulement de l'enrobage extérieur, mais également une biocompatibilité de l'élément enrobé (dans lequel est disposé l'élément actif) afin d'assurer une protection même en cas de contact indirect, par exemple du fait d'une diffusion au travers de l'enrobage, entre l'élément enrobé et le milieu environnant, ou encore en cas de dégradation, blessure, ou fissuration de l'enrobage.

Pour répondre à de telles normes, le document EP 1903 000 A2 propose la réalisation d'un élément actif sur un substrat de silicium dopé au bore ou de borosilicate, puis une encapsulation de l'élément actif dans une cavité réalisée en reportant et en scellant hermétiquement un capot, également composé de silicium ou de borosilicate, sur le substrat. Afin de pouvoir accéder électriquement à l'élément actif, des liaisons électriques entre l'élément actif et l'extérieur de la cavité sont formées par des vias conducteurs réalisés à travers le substrat, les extrémités de ces vias étant reliés à des plots de contacts électriques réalisés de chaque côté du substrat (c'est-à-dire à la fois dans la cavité pour être relié électriquement à l'élément actif, et sur la face arrière du substrat).

Le dispositif médical réalisé doit être hermétique, c'est-à-dire résistant non seulement à la pénétration des liquides (étanchéité), mais également à celle des gaz, ce qui suppose un taux de fuite vis-à-vis de la cavité dans le dispositif médical qui soit parfaitement contrôlé et infinitésimal.

Un moyen pour mesurer l'absence ou la présence de fuite consiste à mesurer une déflection de la membrane du capot en fonction d'un différentiel de pression entre l'intérieur de la cavité et le milieu extérieur. Compte tenu du matériau de la membrane du capot, de ses dimensions et du différentiel de pression à ses bornes, il est possible de calculer la déflection de la membrane. A l'inverse, compte tenu de la pression externe de la membrane (dimensions et matériau), il est possible d'en déduire la pression interne.

La solution proposée par le document EP 1 903 000 A2 permet de répondre à ces normes relatives à la double protection des éléments actifs destinés à être implantés. Toutefois, cette solution implique la réalisation d'un empilement (substrat + capot) en silicium ou en quartz intégrant à la fois des cavités et des vias traversant en présence d'un capot usiné et scellé hermétiquement au substrat, ce qui est complexe et coûteux à mettre en oeuvre.

Par ailleurs, dans les domaines autres que médical, il est connu de packager un ou plusieurs éléments actifs (tels que des capteurs inertiels, sonores, de températures, de rayonnements, ...) dans des cavités fermées. La connexion électrique de ces éléments actifs est réalisée de l'intérieur de la cavité vers l'extérieur de celle-ci par des TSV (« Through Silicon Via » ou vias traversants). Cette technologie de reprise de contacts nécessite comme précédemment un nombre d'étapes important, ce procédé étant donc coûteux à mettre en oeuvre.

### EXPOSÉ DE L'INVENTION

Un but de la présente invention est de proposer un composant intégrant un ou plusieurs éléments actifs et/ou passifs encapsulés dans une cavité et pouvant être contactés électriquement depuis l'extérieur de la cavité, et qui soit moins complexe et moins coûteux à réaliser que les composants de l'art antérieur.

L'invention peut notamment correspondre à un composant biocompatible apte à être implanté dans un corps, ou une partie d'un corps, humain ou animal, répondant aux exigences de protection du ou des éléments actifs du composant, dont au moins l'extérieur est biocompatible.

Pour cela, il est proposé un composant comportant au moins un élément actif encapsulé hermétiquement dans au moins une cavité formée entre au moins un support et au moins un capot, dans lequel le support et le capot sont en au moins un matériau électriquement conducteur et sont isolés électriquement l'un par rapport à l'autre, et comprenant une première liaison électrique entre l'élément actif et le support, et une deuxième liaison électrique, distincte de la première liaison électrique, entre l'élément actif et le capot.

L'invention concerne en outre un composant comportant au moins un élément actif encapsulé hermétiquement dans au moins une cavité formée entre au moins un support et au moins un capot, dans lequel le support et le capot sont en au moins un matériau électriquement conducteur et sont isolés électriquement l'un par rapport à l'autre, et comprenant une première liaison électrique entre l'élément actif et le support, et une deuxième liaison électrique, distincte de la première liaison électrique, entre l'élément actif et le capot, et dans lequel :
- l'élément actif est solidarisé au support par l'intermédiaire d'au moins une couche diélectrique disposée entre le support et l'élément actif, et entre le support et le capot ;
- la deuxième liaison électrique comporte au moins une deuxième portion de matériau électriquement conducteur reliée électriquement au capot, disposée sur la couche diélectrique et en contact électriquement avec un cordon de scellement électriquement conducteur assurant une solidarisation hermétique du capot au support.

Ainsi, avec un tel composant, la totalité, ou la quasi-totalité, de la surface extérieure du support et du capot peut servir à former des accès électriques à l'élément actif encapsulé. De plus, avec un tel composant, il n'est pas nécessaire de réaliser des vias conducteurs à travers le support et/ou le capot étant donné que l'ensemble du support électriquement conducteur et l'ensemble du capot électriquement conducteur forment des liaisons électriques avec l'élément actif.

Il est également décrit un composant biocompatible apte à être implanté dans au moins une partie d'un corps humain ou animal, comportant au moins un élément actif encapsulé hermétiquement dans au moins une cavité formée entre au moins un support et au moins un capot, dans lequel le support et le capot est en un matériau électriquement conducteur, l'élément actif étant relié électriquement au support et au capot par deux liaisons électriques distinctes.

Il est également proposé un composant biocompatible apte à être implanté dans au moins une partie d'un corps humain ou animal, comportant au moins un élément actif encapsulé hermétiquement dans au moins une cavité formée entre au moins un support et au moins un capot, dans lequel le support et le capot comportent chacun au moins une couche entièrement, ou intégralement, composée de matériau électriquement conducteur, l'élément actif étant relié électriquement aux couches composées de matériau électriquement conducteur du support et du capot.

La totalité, ou la quasi-totalité, de la surface de la face avant et de la face arrière du composant, c'est-à-dire la totalité ou la quasi-totalité de la surface de la face avant du capot et de la face arrière du support, peut être électriquement conductrice. La totalité, ou la quasi-totalité, de la surface des faces latérales du capot et du support peut également être électriquement conductrice.

L'élément actif peut comporter au moins un circuit électronique intégré et/ou un dispositif de type MEMS/NEMS et/ou un capteur de mesure d'au moins un paramètre physiologique. De manière générale, le composant peut comporter n'importe quel type d'élément actif, et éventuellement un ou plusieurs éléments, ou composants, passifs tels qu'une capacité par exemple de découplage ou une diode de protection, également encapsulés dans la cavité et éventuellement reliés électriquement au support et/ou au capot.

Le matériau du support et/ou du capot peut être du semi-conducteur, par exemple du silicium, dopé dont la résistivité est comprise entre environ 0,5 mOhm.cm et 20 mOhm.cm (ou plus généralement dont la résistivité est adaptée à l'application envisagée) et/ou du métal.

L'élément actif peut être solidarisé au support par l'intermédiaire d'au moins une couche diélectrique disposée entre le support et l'élément actif, et entre le support et le capot. Une telle couche diélectrique permet d'assurer une isolation électrique entre l'élément actif et le support, ainsi qu'une isolation électrique entre le support et le capot qui sont électriquement conducteurs.

Dans ce cas, la première liaison électrique peut comporter au moins une première portion de matériau électriquement conducteur en contact avec le support et disposée au moins dans une ouverture, ou trou, formée à travers la couche diélectrique, et/ou dans lequel la deuxième liaison électrique peut comporter au moins une deuxième portion de matériau électriquement conducteur reliée électriquement au capot et disposée sur la couche diélectrique. La première portion de matériau électriquement conducteur peut être reliée électriquement à l'élément actif par au moins un fil électrique.

La deuxième portion de matériau électriquement conducteur peut être en contact électriquement avec un cordon de scellement, par exemple métallique, électriquement conducteur assurant une solidarisation hermétique du capot au support.

L'élément actif peut être solidarisé au support par des micro-plots (par exemple des microbilles de matériau fusible, des micro-piliers électriquement conducteurs, etc.) assurant en outre la liaison électrique entre l'élément actif et le support et/ou la liaison électrique entre l'élément actif et le capot), ou l'élément actif peut être collé sur la couche diélectrique et la première et/ou la deuxième liaison électrique peut comporter au moins un fil électrique.

Le capot peut comporter un substrat électriquement conducteur scellé hermétiquement au support, ou le capot peut comporter au moins une couche mince électriquement conductrice. Une telle couche mince électriquement conductrice peut comporter une épaisseur comprise entre environ 1 µm et 10 µm, ou entre environ 1 µm et 5 µm, ou plus généralement inférieure à environ 10 µm.

Le composant peut comporter en outre, lorsque le capot comporte une couche mince électriquement conductrice, par exemple composée d'un matériau métallique de préférence biocompatible, au moins une couche mince diélectrique disposée contre la couche mince électriquement conductrice du capot et entre le support et la couche mince électriquement conductrice du capot, la deuxième liaison électrique pouvant traverser la couche mince diélectrique via au moins une ouverture. Ainsi, la couche mince diélectrique assure dans ce cas une isolation électrique entre le support et le capot métallique.

Le composant peut comporter plusieurs éléments actifs encapsulés hermétiquement et individuellement dans des cavités formées entre le support et plusieurs capots isolés électriquement les uns des autres, chacun des éléments actifs pouvant être relié électriquement à l'un des capots et au support. Le composant peut également comporter un ou plusieurs éléments passifs, par exemple des résistances, des capacités ou des inductances, reliés électriquement au capot et/ou au support de la même façon que les éléments actifs.

Le composant peut comporter plusieurs éléments actifs encapsulés hermétiquement et individuellement dans des cavités formées entre le capot, ou les capots, et plusieurs supports isolés électriquement les uns des autres, chacun des éléments actifs étant relié électriquement à l'un des supports électriquement conducteurs et au capot ou à l'un des capots.

Le support et le capot peuvent être enrobés dans un matériau biocompatible, par exemple du silicone, du parylène, une résine époxyde, de l'oxyde de silicium, de l'oxyde tantale et/ou de l'oxyde de titane, réalisé par exemple sous la forme une couche mince réalisée par dépôt atomique (ALD).

L'invention concerne également un dispositif médical implantable, ou apte à être implanté, dans un corps, ou au moins une partie d'un corps, humain ou animal, comportant au moins un composant tel que défini ci-dessus.

Il est également proposé un procédé de réalisation d'un composant comportant au moins les étapes de :
- réalisation d'au moins un élément actif,
- réalisation d'une première liaison électrique entre l'élément actif et au moins un support et d'une deuxième liaison électrique, distincte de la première liaison électrique, entre l'élément actif et au moins un capot,
- encapsulation hermétique de l'élément actif dans au moins une cavité formée entre le support et le capot,
dans lequel le support et le capot sont en un matériau électriquement conducteur et sont isolés électriquement l'un par rapport à l'autre.

L'invention concerne en outre un procédé de réalisation d'un composant comportant au moins les étapes de :
- réalisation d'au moins un élément actif,
- réalisation d'une première liaison électrique entre l'élément actif et au moins un support et d'une deuxième liaison électrique, distincte de la première liaison électrique, entre l'élément actif et au moins un capot,
- encàpsulation hermétique de l'élément actif dans au moins une cavité formée entre le support et le capot,
   et dans lequel :
- le support et le capot sont en un matériau électriquement conducteur et sont isolés électriquement l'un par rapport à l'autre ;
- l'élément actif est solidarisé au support par l'intermédiaire d'au moins une couche diélectrique disposée entre le support et l'élément actif, et entre le support et le capot ;
- la deuxième liaison électrique comporte au moins une deuxième portion de matériau électriquement conducteur reliée électriquement au capot, disposée sur la couche diélectrique et en contact électriquement avec un cordon de scellement électriquement conducteur assurant une solidarisation hermétique du capot au support.

Le support et le capot peuvent être solidarisés l'un à l'autre par wafer bonding avec une activation plasma. Dans ce cas, le support et le capot font parties de deux substrats solidarisés l'un à l'autre, l'encapsulation de l'élément actif étant donc réalisée à l'échelle des wafers, ou des substrats. Cette technique est avantageuse car la solidarisation est alors réalisée à basse température et est biocompatible.

### BRÈVE DESCRIPTION DES DESSINS

La présente invention sera mieux comprise à la lecture de la description d'exemples de réalisation donnés à titre purement indicatif et nullement limitatif en faisant référence aux dessins annexés sur lesquels :
- les figures 1A à 6 et 8 représentent des composants, objets de la présente invention, selon différents modes de réalisation,
- les figures 7A à 7H représentent les étapes d'un procédé de réalisation d'un composant, objet de la présente invention, selon un mode de réalisation particulier,
- la figure 9 représente un exemple de dispositif médical implantable, objet de la présente invention, selon un mode de réalisation particulier.

Des parties identiques, similaires ou équivalentes des différentes figures décrites ci-après portent les mêmes références numériques de façon à faciliter le passage d'une figure à l'autre.

Les différentes parties représentées sur les figures ne le sont pas nécessairement selon une échelle uniforme, pour rendre les figures plus lisibles.

Les différentes possibilités (variantes et modes de réalisation) doivent être comprises comme n'étant pas exclusives les unes des autres et peuvent se combiner entre elles.

### EXPOSÉ DÉTAILLÉ DE MODES DE RÉALISATION PARTICULIERS

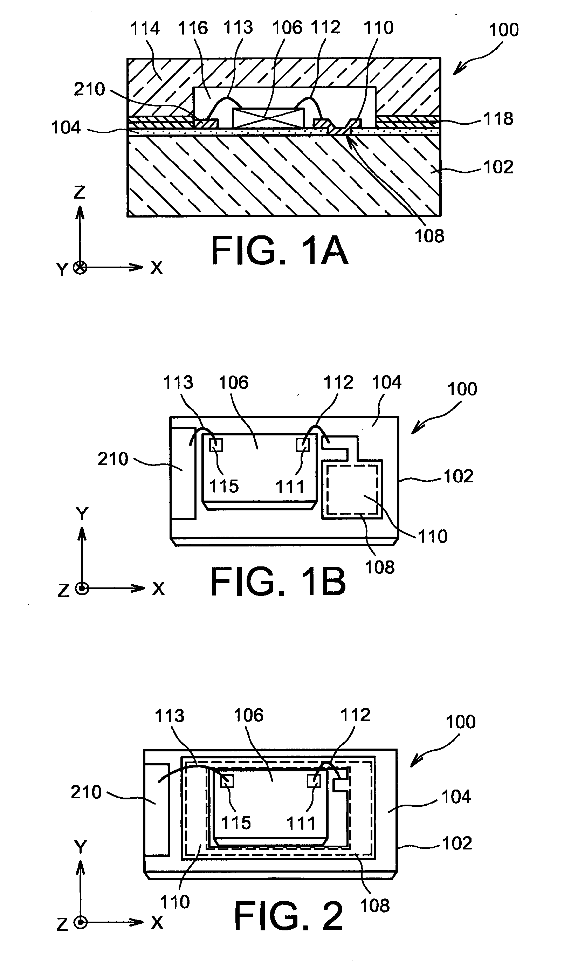

On se réfère tout d'abord aux figures 1A et 1B qui représentent respectivement une vue en coupe de profil et une vue de dessus d'un composant 100 selon un premier mode de réalisation.

Le composant 100 comporte un support 102 composé de semi-conducteur, ici du silicium, fortement dopé, c'est-à-dire tel que le support 102 soit électriquement conducteur. Le support 102 est ici un substrat. Le dopage peut être du type N ou P et tel que le semi-conducteur du support 102 présente une faible résistivité, par exemple comprise entre environ 0,5 mΩ.cm et 20 mΩ.cm. Une résistivité de type P ou N de 0,5 mΩ.cm peut être obtenue en réalisant un dopage d'environ 2.10²⁰ at/cm³. Une résistivité de type P de 20 mΩ.cm peut être obtenue par exemple en réalisant un dopage d'environ 3.10¹⁸ at/cm³. Enfin, une résistivité de type N de 20 mΩ.cm peut être obtenue en réalisant par exemple un dopage d'environ 1.10¹⁸ at/cm³.

Dans ce premier mode de réalisation, le support 102 a par exemple une épaisseur (dimension selon l'axe Z) comprise entre environ 50 µm et 500 µm, ou supérieure à environ 50 µm, une longueur (dimension selon l'axe X) comprise entre environ 0,5 mm et 10 mm, et une largeur (dimension selon l'axe Y) comprise entre environ 0,5 mm et 5 mm.

La face avant du support 102 est recouverte par une couche diélectrique 104. La couche diélectrique 104 est ici une couche de passivation de type organique ou minéral, par exemple composée de SiN et/ou de SiO₂ et/ou de SiON, dont l'épaisseur est comprise entre environ 100 nm et 5 µm.

Un élément actif 106, par exemple un capteur, un MEMS ou NEMS, ou encore un circuit électronique intégré de type ASIC ou FPGA, est solidarisé sur la couche diélectrique 104. La couche diélectrique 104 assure une isolation électrique entre l'élément actif 106 et le support 102 qui est électriquement conducteur en raison du dopage réalisé. Dans le premier mode de réalisation représenté sur les figures 1A et 1B, l'élément actif 106 est collé contre la couche diélectrique 104.

La couche diélectrique 104 comporte une ouverture 108, ou trou, formant un accès au support 102 depuis le côté où se trouvent la couche diélectrique 104 et l'élément actif 106. Une portion de matériau électriquement conducteur 110, par exemple composée de métal, est disposée dans cette ouverture 108. Un fil électrique 112, par exemple composé d'or, relie électriquement l'élément actif 106 à la portion de matériau électriquement conducteur 110. Ainsi, l'élément actif 106 est relié électriquement, via au moins un plot de connexion électrique 111 présent sur la face avant de l'élément actif 106, c'est-à-dire la face de l'élément actif 106 opposée à celle en contact avec la couche diélectrique 104, au support 102 par une première liaison électrique formée par la portion 110 et le fil électrique 112. La portion 110 est réalisée telle qu'elle permette d'assurer un bon contact ohmique avec le support 102 et de réaliser une connexion électrique (par exemple une soudure) avec le fil électrique 112. Pour cela, la portion 110 est par exemple composée de Ti et/ou de WN et/ou de Ni et/ou d'Al et/ou de Cu et/ou d'Au, et a ici une épaisseur comprise entre environ 100 nm et 5 µm.

Un capot 114, formé ici par un substrat de semi-conducteur, tel que du silicium, dopé, est solidarisé au support 102 en formant une cavité hermétique 116 dans laquelle l'élément actif 106 est encapsulé. Un ou plusieurs composants passifs, tels qu'une capacité de découplage ou une diode de protection, peuvent également être réalisés et encapsulés dans la cavité 116. Cette cavité 116 est en partie formée par gravure dans le capot 114. La solidarisation hermétique du capot 114 au support 102 est réalisée par un cordon de scellement 118 composé de matériau électriquement conducteur, par exemple de l'or ou un alliage biocompatible à base d'or, formé sur la couche diélectrique 104. Ce cordon de scellement 118 est par exemple obtenu par thermocompression entre au moins une portion métallique formée au préalable sur la couche diélectrique 104 (par exemple issue de la même couche de matériau que celle utilisée pour réaliser la portion 110) et au moins une portion métallique formée sur la face du capot 114 destinée à se trouver du côté du support 102, en périphérie de la cavité 116. Le cordon de scellement 118 peut également être obtenu par inter-diffusion d'un cordon composé d'or avec le semi-conducteur du capot.

Une deuxième liaison électrique, distincte de la première liaison électrique, assure la connexion électrique de l'élément actif 106 avec le capot 114. Pour cela, un deuxième fil électrique 113 relie l'élément actif 106, via au moins autre plot de connexion 115 présent sur la face avant de l'élément actif 106 auquel est relié le fil électrique 113, à une deuxième portion de matériau électriquement conducteur 210, par exemple de nature similaire au matériau de la portion 110, réalisée sur la couche diélectrique 104, à côté de l'élément actif 106. Cette deuxième portion de matériau électriquement conducteur 210 est en contact électriquement avec le cordon de scellement 118 qui est électriquement conducteur. Or, étant donné que le cordon de scellement 118 est également en contact électrique avec le capot 114, l'élément actif 106 est donc bien relié électriquement au capot 114.

Ainsi, avec le composant 100, l'élément actif 106 est accessible électriquement, par exemple pour envoyer à l'élément actif 106 des signaux de commande et/ou recevoir des signaux de sortie délivrés par l'élément actif 106, via le support 102 et le capot 114 qui sont en matériaux électriquement conducteurs. L'élément actif 106 comporte donc deux liaisons électriques accessibles indépendamment par l'intermédiaire du capot 114 et du support 102 qui sont en matériaux électriquement conducteurs. Ces accès électriques à l'élément actif 106 peuvent se faire d'une part depuis la face arrière du support 102 et/ou depuis les faces latérales (celles se trouvant dans des plans parallèles au plan (Y, Z)) du support 102, et d'autre part depuis la face avant du capot 114 et/ou depuis les faces latérales du capot 114. L'isolation électrique entre le support 102 et le capot 114 est assurée par la couche diélectrique 104.

Afin de réduire au maximum la résistance d'accès au support 102, les dimensions de l'ouverture 108 sont maximisées afin d'offrir une grande surface de contact entre la portion de matériau électriquement conducteur 110 et le support 102. Cette surface de contact est avantageusement telle que la résistance d'accès au support soit inférieure à environ 1 Ohm, et par exemple comprise entre environ 0,05 mm² et 5 mm².

De même, afin de réduire au maximum la résistance d'accès au capot 114, les dimensions de la surface de contact entre la portion 210 et le cordon de scellement 118 peuvent être maximisées, comme pour la surface de contact entre la portion 110 et le support 102 du composant 100.

Dans le premier mode de réalisation représenté sur les figures 1A et 1B, l'ouverture 108, et donc la partie de la portion 110 se trouvant en contact avec le support 102, a une forme rectangulaire (la figure 1B correspond à une vue de dessus du composant 100 sans la présence du capot 114 et du cordon de scellement 118). De plus, l'ouverture 108 est réalisée à côté de l'élément actif 106. Dans une variante représentée sur la figure 2, l'ouverture 108 est réalisée sous la forme d'une tranchée entourant l'élément actif 106, la portion de matériau électriquement conducteur 110 étant disposée au moins en partie dans l'ouverture 108, autour de l'élément actif 106.

Dans un deuxième mode de réalisation, il est possible de réaliser la portion 110 telle qu'elle soit directement reliée à l'élément actif 106, sans avoir à utiliser un fil électrique. Dans ce cas, l'élément 106 est par exemple reporté par flip-chip (solidarisé par microbilles) sur des métallisations reliées électriquement aux portions de matériau électriquement conducteur 110 et 210.

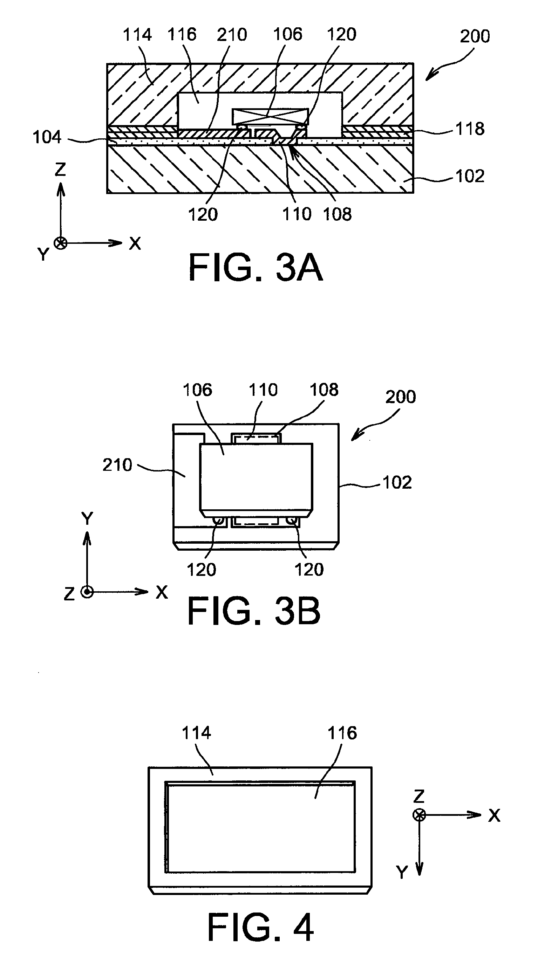

Les figures 3A et 3B représentent un composant 200 selon un deuxième mode de réalisation. Comme pour le composant 100 précédemment décrit, le composant 200 comporte le support 102 électriquement conducteur, la couche diélectrique 104, l'élément actif 106, l'ouverture 108 réalisée à travers la couche diélectrique 104, la portion de matériau électriquement conducteur 110, le capot électriquement conducteur 114, le cordon de scellement 118 et la portion électriquement conductrice 210. Toutefois, l'élément actif 106 est ici directement soudé, au niveau de sa face avant qui comporte au moins deux plots de contact électrique, à des portions de matériau métallique, dont les portions 110 et 210, par « flip-chip », c'est-à-dire via des micro-plots 120 de dimensions micrométriques ou nanométriques et par exemple composées d'au moins un matériau fusible. Ces micro-plots 120 peuvent correspondre à des microbilles, des piliers composés de cuivre, des micro-inserts ou encore tout type de micro-éléments composés par exemple de matériau fusible. Dans l'exemple représenté sur les figures 3A et 3B, un seul des micro-plots 120 est relié électriquement à la portion de matériau électriquement conducteur 110 qui est disposée sous l'élément actif 106 (l'ouverture 108 étant réalisée ici sous l'élément actif 106), ce micro-plot 120 assurant une liaison électrique et une partie de la liaison mécanique entre l'élément actif 106 et le support 102 via la portion 110. Un autre micro-plot 120 est relié électriquement à la portion de matériau électriquement conducteur 210 et assure donc, avec la portion 210 et le cordon de scellement 118, la liaison électrique entre l'élément actif 106 et le capot électriquement conducteur 114. D'autres micro-plots, non représentés sur les figures 3A et 3B, assurent le reste de la liaison mécanique entre l'élément actif 106 et le support 102 via des portions de matériau métallique, par exemple de nature similaire au matériau des portions 110 et 210, réalisées sur la couche diélectrique 104 (la couche diélectrique 104 isolant électriquement ces portions métalliques du support 102).

Dans ce deuxième mode de réalisation, l'ouverture 108 n'est pas nécessairement réalisée sous l'emplacement de l'élément actif 106. Il est en effet possible que l'ouverture 108 soit réalisée à côté et/ou autour de l'élément actif 106, comme sur les exemples des figures 1A, 1B et 2 précédemment décrits. Dans ce cas, une partie de la portion 110 s'étend en partie sous l'élément actif 106 afin qu'au moins un des micro-plots 120 puisse être solidarisé à cette partie de la portion 110 et ainsi relier électriquement l'élément actif 106 au support 102.

La figure 4 représente une vue de dessous du capot 114 électriquement conducteur.

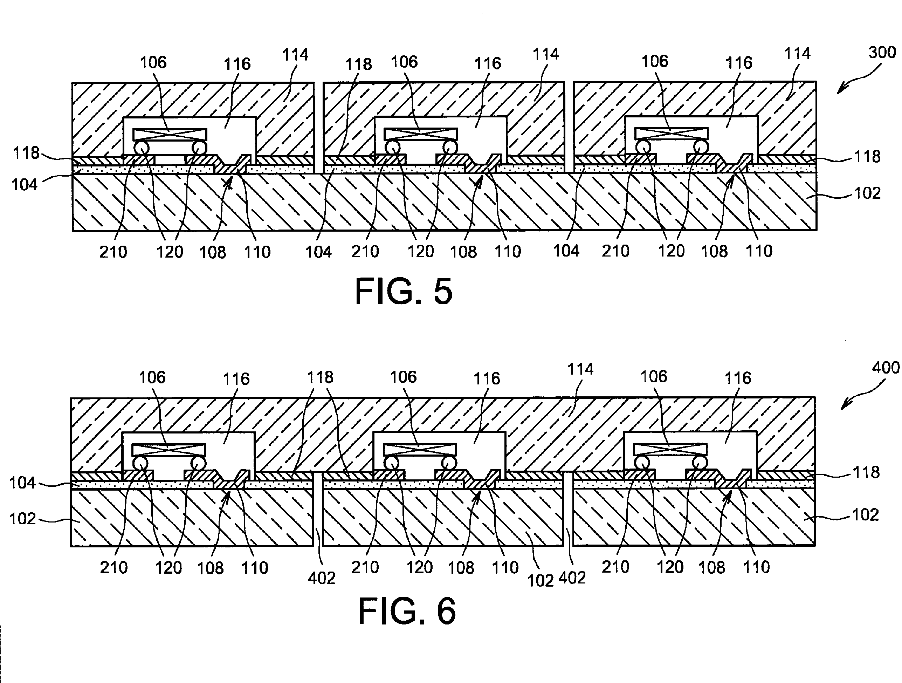

La figure 5 représente un composant 300 selon un troisième mode de réalisation, comportant plusieurs éléments actifs 106 réalisés sur un même support 102 et encapsulés dans des cavités 116 indépendantes les unes des autres. Les éléments actifs 106 du composant 300 sont par exemple similaires à l'élément actif 106 du composant 200 précédemment décrit, c'est-à-dire solidarisés au support 102 via les micro-plots 120 assurant une première connexion électrique au support 102 (cette connexion étant commune pour tous les éléments actifs 106 du fait que l'ensemble du substrat formant le support 102 est électriquement conducteur) et une deuxième connexion électrique à chaque capot 114 électriquement conducteur associé à chacun des éléments actifs 106. Les capots 114 sont réalisés tels qu'ils soient isolés électriquement les uns des autres, assurant ainsi une indépendance des liaisons électriques entre les capots 114 et les éléments actifs 106 encapsulés.

La figure 6 représente un composant 400 selon un quatrième mode de réalisation, comportant plusieurs éléments actifs 106 encapsulés dans des cavités 116 indépendantes les unes des autres. Par rapport au composant 300 précédemment décrit, les éléments actifs 106 sont réalisés sur des supports 102 isolés électriquement les uns des autres (cette isolation étant par exemple obtenue en gravant des tranchées 402 dans un substrat, formant les supports 102, les tranchées 402 traversant également la couche diélectrique 104 et le cordon de scellement 118). Ainsi, les liaisons électriques réalisées entre chaque élément actif 106 et le support 102 sur lequel est solidarisé l'élément actif 106 associé sont indépendantes les unes des autres. De plus, contrairement au composant 300 précédemment décrit, les cavités 116 dans lesquelles les éléments actifs 106 sont encapsulés sont formées par un unique capot 114 (les cavités 116 étant par exemple gravées dans un unique substrat formant le capot 114) solidarisé hermétiquement aux différents supports 102 (la gravure des tranchées 402 pouvant être réalisée après avoir solidarisé le capot 206 au substrat initial non gravé sur lequel les éléments actifs 106 sont réalisés). Les liaisons électriques réalisées entre les éléments actifs 106 et le capot 114 ne sont pas isolées les unes par rapport aux autres.

Dans une variante des deux précédents modes de réalisation, il est également envisageable que les connexions électriques éléments actifs 106 - capot 114 et éléments actifs 106-substrat 102 soient toutes indépendantes.

Dans les composants 300 et 400 précédemment décrits, les éléments actifs 106 sont solidarisés au(x) support(s) 102 par flip-chip, via des microbilles 120. En variante, il est possible que les éléments actifs 106 soient solidarisés au(x) support(s) 102 par exemple par collage sur la couche diélectrique 104, comme le composant 100 précédemment décrit. Dans ce cas, les liaisons électriques éléments actifs - capot et/ou les liaisons électriques éléments actifs - support peuvent être réalisées par des fils électriques comme précédemment décrit. Les composants 300 et 400 peuvent être rendus biocompatibles comme précédemment décrit pour les composants 100 et 200.

Dans tous les modes de réalisation précédemment décrits, la liaison électrique entre l'élément actif 106 et le support 102 est assurée par une portion de matériau électriquement conducteur 110 directement en contact avec le support 102 au niveau de l'ouverture 108 formée à travers la couche diélectrique 104. Afin d'améliorer le contact ohmique entre le support 102 et la portion de matériau électriquement conducteur 110, il est possible de réaliser localement un dopage par implantation ionique dans le support 102, au niveau de l'ouverture 108, la portion de matériau électriquement conducteur 110 étant dans ce cas en contact direct avec cette zone dopée du support 102. Suivant le type d'implantation ionique réalisée (N ou P, et qui est du même type que celui du support 102), celle-ci permet d'améliorer le contact ohmique entre le support 102 et la portion électriquement conductrice 110. Une telle implantation ionique peut également être réalisée dans le capot au niveau des zones de contact avec le cordon de scellement solidarisant le capot au support. Ainsi, on améliore le contact ohmique entre le capot et le cordon de scellement, et donc la qualité de la liaison électrique entre le capot et le ou les éléments actifs.

Lors de la réalisation du composant, après le dépôt du matériau de la portion 110 dans l'ouverture 108, il est possible de réaliser un traitement thermique formant, au niveau de l'interface entre la portion 110 et le support 102, une portion de siliciure améliorant encore le contact électrique entre le support 102 et la portion de matériau électriquement conducteur 110.

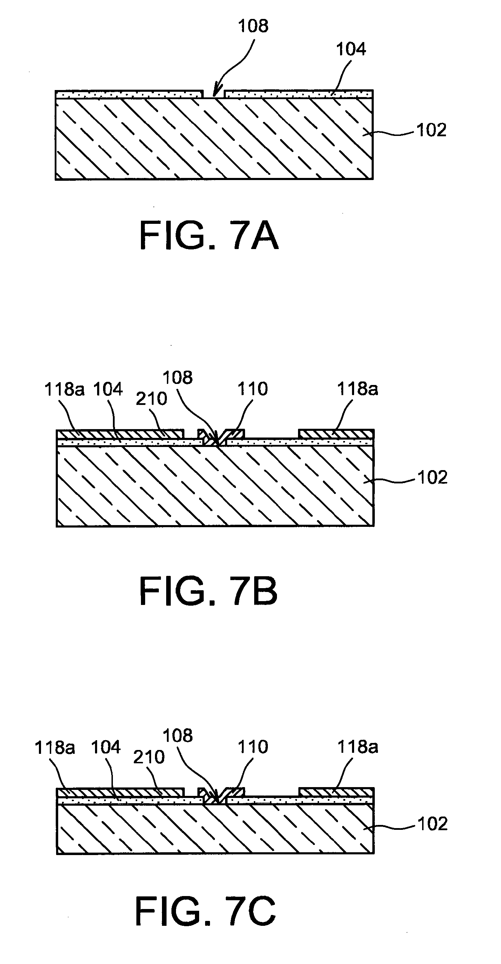

On décrit maintenant, en liaison avec les figures 7A à 7H, un procédé de réalisation d'un composant 500 selon un cinquième mode de réalisation. Bien que l'on décrive, en liaison avec ces figures, la réalisation d'un seul composant 500, ces étapes peuvent correspondre à des étapes de réalisation collective de plusieurs composants, les substrats utilisés pouvant servir à former les capots et les supports de plusieurs composants. Les différents éléments actifs sont alors encapsulés de manière collective dans des cavités indépendantes, les substrats étant ensuite découpés afin de rendre indépendants les composants réalisés.

Dans une première étape, une face avant du support 102, correspondant à un substrat de semi-conducteur fortement dopé (résistivité par exemple comprise entre environ 0,5 mOhm.cm et 20 mOhm.cm), de type N ou P, est recouverte d'une couche de passivation correspondant à la couche diélectrique 104. Suivant la nature du matériau de la couche diélectrique 104, par exemple composée de SiO₂, de SiN ou de SiON, celle-ci est par exemple réalisée via une oxydation thermique ou un dépôt sur la face avant du support 102. Une ouverture 108, destinée à former une zones d'accès au support 102 pour la future connexion électrique de l'élément actif, est ensuite réalisée à travers la couche diélectrique 104, par exemple en mettant en oeuvre des étapes de photolithographie et de gravure (figure 7A).

Comme représenté sur la figure 7B, une couche de matériau électriquement conducteur, ici du métal tel que de l'or ou un alliage d'or et d'étain, est déposée sur la couche diélectrique 104 (directement ou via une éventuelle couche d'accroche et/ou une éventuelle couche barrière) et dans l'ouverture 108 afin d'obtenir un contact ohmique entre le support 102 et cette couche électriquement conductrice. Cette couche est par exemple réalisée via un dépôt électrolytique, un dépôt plasma (par exemple de type PECVD, c'est-à-dire un dépôt chimique en phase vapeur assisté par plasma), ou encore un dépôt par évaporation ou par PVD (dépôt physique en phase vapeur). Cette couche est ensuite structurée par gravure afin de former les portions de matériau électriquement conducteur 110 et 210 destinées à relier électriquement l'élément actif au support 102 et au capot 114, ainsi qu'une partie 118a du cordon de scellement 118. En variante, il est possible de réaliser le cordon de scellement indépendamment des portions de matériau électriquement conducteur 110, 210, via la mise en oeuvre d'étapes de dépôt et de gravure distinctes, par exemple lorsque la partie 118a du cordon de scellement 118 est composée d'un matériau différent et/ou a une épaisseur différente des portions électriquement conductrices 110, 210 à réaliser. De même, lorsque le composant biocompatible comporte des plots métalliques d'accueil pour des câbles ou des microbilles fusibles, ces éléments peuvent être réalisés indépendamment de la portion 110 et de la partie 208a du cordon de scellement 208.

Afin d'améliorer le contact ohmique entre la portion 110 et le support 102, on peut mettre en oeuvre un traitement thermique permettant de diffuser des espèces métalliques (provenant de la portion 110) dans le support 102, au niveau de l'ouverture 108, formant ainsi un siliciure au niveau du contact avec la portion 110.

Lorsque le support 102 est trop épais, il est possible de l'amincir, par exemple via un meulage (« grinding ») et/ou un polissage mécano-chimique au niveau de sa face arrière, c'est-à-dire la face opposée à celle sur laquelle se trouve la couche diélectrique 104 (voir figure 7C).

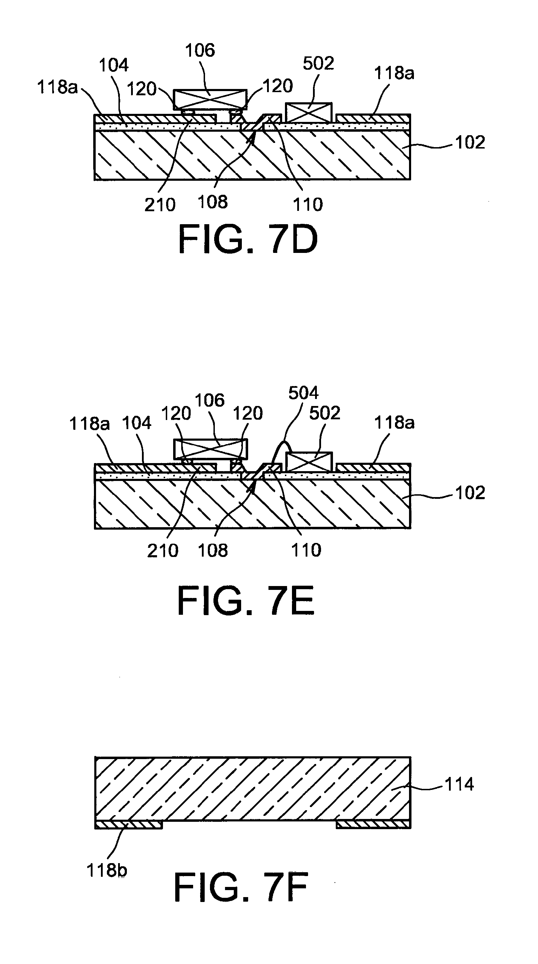

Les différents éléments électroniques (par exemple des éléments actifs tels que des circuits électroniques et/ou des NEMS ou MEMS, et/ou des composants passifs) du composant biocompatible 500 sont ensuite reportés sur le support 102. Sur l'exemple de la figure 7D, l'élément actif 106 est solidarisé par flip-chip, via les micro-plots 114 (ici des microbilles), aux portions métalliques formées sur le support 102. L'une des microbilles 120 est reliée électriquement à la portion métallique 110, assurant ainsi la liaison électrique entre l'élément actif 106 et le support 102, et au moins une autre des microbilles 120 est reliée électriquement à la portion 210 qui se confond avec la partie 118a du cordon de scellement, assurant la liaison électrique entre l'élément actif 106 et le cordon de scellement 118 qui servira à solidariser le capot 114 en matériau électriquement conducteur au support 102. Un élément passif 502 est également reporté sur le support 102, via un collage sur la couche diélectrique 104.

Les éventuels câblages filaires des éléments précédemment reportés sur le support 102 sont alors réalisés. Sur la figure 7E, l'élément 502 est relié électriquement à la portion 110 par un fil 504 (les autres câblages filaires reliés à l'élément 502 ne sont pas représentés).

Parallèlement aux étapes précédemment décrites en liaison avec les figures 7A à 7E, on prépare le capot 114 destiné à être reporté sur le support 102.

Pour cela, on réalise tout d'abord un dépôt et une structuration d'une couche métallique, par exemple composée d'or ou d'un alliage d'or et d'étain, sur un substrat de semi-conducteur fortement dopé (tel qu'il soit électriquement conducteur), telle que les portions restantes 118b de la couche métallique soient destinées à former une partie du cordon de scellement 118 (figure 7F). La couche métallique peut être formée par voie électrolytique, par un dépôt plasma ou par un dépôt par évaporation. Un traitement thermique peut également être mis en oeuvre afin de diffuser dans le capot 114 des espèces métalliques provenant des portions 118b, et améliorer ainsi le contact ohmique entre le capot 114 et ces portions 118b.

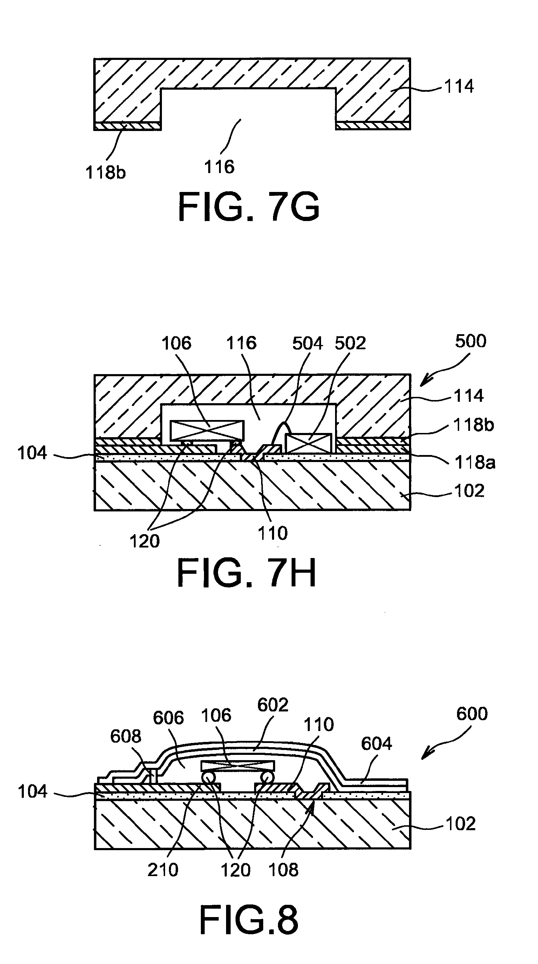

Le substrat formant le capot 114 est ensuite gravé afin de former la cavité 116 dans laquelle les éléments électriques (élément actif 106 et élément passif 502) du composant 500 seront encapsulés (figure 7G). La cavité 116 est par exemple réalisée par une gravure chimique à partir d'une solution de type KOH ou TMAH, ou avantageusement par une gravure plasma de type DRIE (gravure ionique réactive profonde) permettant d'obtenir des flancs de la cavité 116 qui soient bien perpendiculaires par rapport aux faces principales du substrat à partir duquel est réalisé le capot 114. En fonction de la profondeur de la cavité 116, la gravure du capot 114 est réalisée à travers un masque de résine photosensible et/ou un masque dur par exemple en oxyde de silicium.

Enfin, comme représenté sur la figure 7H, le capot 114 est solidarisé au support 102 via une solidarisation, par exemple réalisée par fusion ou thermocompression, des portions 118a avec les portions 118b, formant le cordon de scellement 118. Cette solidarisation peut se faire à l'échelle du wafer, formant simultanément plusieurs composants biocompatibles via une encapsulation collective des éléments réalisés sur le support 102, soit à l'échelle de la puce, c'est-à-dire en solidarisant uniquement le capot 114 du composant 500 au support 102. Dans le cas d'une encapsulation collective formant plusieurs composants biocompatibles, les substrats utilisés pour former le ou les capots et le ou les supports peuvent être découpés afin d'obtenir les dispositifs biocompatibles indépendants.

Dans les exemples précédemment décrits, le capot 114 correspond à un substrat reporté et solidarisé au support. En variante, il est possible que le capot du composant corresponde à une ou plusieurs couches minces. Pour cela, lors de l'encapsulation de l'élément actif du composant, au lieu de reporter sur le support un substrat dans lequel la cavité a été gravée, on dépose sur le support 102 une couche sacrificielle composée d'un matériau apte à être gravé sélectivement par rapport aux autres matériaux qui seront présents dans la cavité (matériaux du capot, de l'élément actif, de la couche diélectrique 104, etc.). Cette couche sacrificielle est structurée afin que la portion restante (ou les portions restantes dans le cas d'une encapsulation collective de plusieurs éléments actifs dans différentes cavités), dont l'épaisseur est par exemple comprise entre environ 1 µm et 100 µm correspond au volume de la cavité à réaliser pour intégrer le ou les éléments actifs / passifs. Une ou plusieurs couches minces (dont l'épaisseur est par exemple comprise entre environ 1 µm et 5 µm), par exemple composées de SiO₂ et/ou de SiN, sont ensuite déposées en recouvrant le support (notamment la couche diélectrique recouvrant le support) et la portion restante de la couche sacrificielle. Une ou plusieurs ouvertures, dont le diamètre peut être compris entre environ 1 µm et 10 µm, sont ensuite réalisées à travers les couches minces, formant ainsi un ou plusieurs accès à la portion de matériau sacrificiel. La portion de matériau sacrificiel est ensuite éliminée par gravure à travers les ouvertures réalisées précédemment. La ou les couches minces diélectriques sont ensuite recouvertes par une ou plusieurs couches minces électriquement conductrices, avantageusement composées de métal et d'épaisseur comprise entre environ 1 µm et 10 µm, formant ainsi le capot en matériau électriquement conducteur et bouchant les ouvertures précédemment réalisées dans la ou les couches minces diélectriques. Comme dans les exemples de réalisation précédemment décrits, l'élément actif est relié électriquement au support et au capot.

Un exemple de réalisation d'un tel composant biocompatible 600, comportant un capot formé par une couche mince, est représenté sur la figure 8. Dans cet exemple, une première couche mince diélectrique 602, dont l'épaisseur est comprise entre environ 1 µm et 5 µm et composée de SiO₂ et/ou de SiN, forme la paroi supérieure interne de la cavité 606 dans laquelle est encapsulé l'élément actif 106. Le capot correspond ici à une couche mince électriquement conductrice 604, dont l'épaisseur est comprise entre environ 1 µ et 10 µm et par exemple composé de métal tel que du Cu et/ou du Ni et/ou de l'Al et/ou de l'Au, recouvrant la couche mince diélectrique 602.

L'élément actif 106 (dont l'épaisseur est avantageusement inférieure ou égale à environ 50 µm de façon à pouvoir être encapsulé par couche mince) est solidarisé au support 102 (qui est recouvert par la couche diélectrique 104) par les micro-plots 120, dont au moins une est solidarisée à la portion de matériau électriquement conducteur 110 reliée électriquement au support 102. Au moins une autre des microbilles 120 est solidarisée à la portion de matériau électriquement conducteur 210 qui est reliée électriquement au capot 604 via une autre portion de matériau électriquement conducteur 608 qui forme un lien métallique conducteur entre la portion 201 et le capot 604 en traversant la couche mince diélectrique 602, au niveau du bord de la cavité 606 afin que ce lien ne soit pas gênant vis-à-vis des éléments présents dans la cavité 606.

Le composant décrit dans les différents modes de réalisation peut s'appliquer à tous les systèmes nécessitant une encapsulation, par exemple sous vide, d'un élément actif, comme par exemple dans le domaine des MEMS/NEMS (Microsystème / Nanosystème Electromécanique), par exemple des résonateurs ou des commutateurs, des MOEMS (Microsystème Opto-Electromécanique) ou encore des détecteurs infrarouges tels que des bolomètres non refroidis.

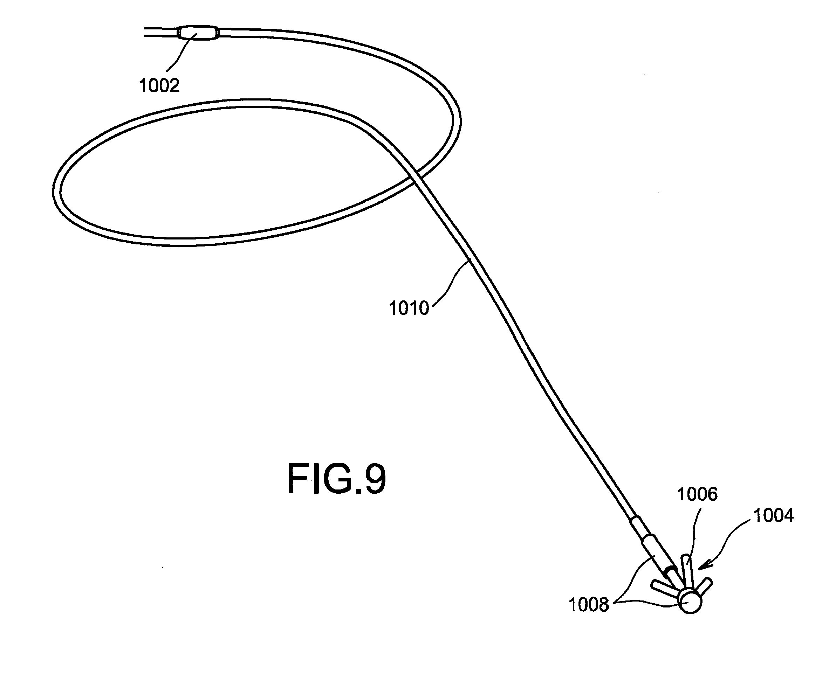

Le composant est avantageusement utilisé pour la réalisation d'un dispositif médical implantable dans un corps, ou une partie d'un corps, humain ou animal. Un exemple d'un tel dispositif médical 1000 est par exemple représenté sur la figure 9. Dans cet exemple, le dispositif médical 1000 est une sonde de stimulation cardiaque. Elle comporte une première extrémité au niveau de laquelle se trouve un connecteur 1002 et une deuxième extrémité au niveau de laquelle se trouve un composant biocompatible 1004 tel que décrit précédemment. Un mécanisme de fixation 1006 et des électrodes 1008 sont également présents à cette deuxième extrémité. Un corps composé de matériau conducteur enrobé dans un matériau isolant biocompatible relie les deux extrémités de la sonde 1000. Dans une telle sonde, l'élément actif du composant 1004 peut correspondre à un capteur de pression sanguine (par exemple de type à membrane souple avec jauges de contrainte ou capacité variable), ou encore un accéléromètre (par exemple piézoélectrique ou de type MEMS) destiné à mesurer une accélération endocardiaque. Les liaisons électriques du capot et du support du composant sont reliées directement ou indirectement à des micro-câbles ou micro-fils eux-mêmes reliés aux deux extrémités de la sonde 1000. Dans une telle sonde, le capot est par exemple relié électriquement à l'électrode proximale et le support est par exemple relié à l'électrode distale, ou inversement.

## Revendications

1. Composant (100 - 600) comportant au moins un élément actif (106) encapsulé hermétiquement dans au moins une cavité (116, 606) formée entre au moins un support (102) et au moins un capot (114, 604), dans lequel le support (102) et le capot (114, 604) sont en au moins un matériau électriquement conducteur et sont isolés électriquement l'un par rapport à l'autre, et comprenant une première liaison électrique entre l'élément actif (106) et le support (102), et une deuxième liaison électrique, distincte de la première liaison électrique, entre l'élément actif (106) et le capot (114, 604), et dans lequel :
- l'élément actif (106) est solidarisé au support (102) par l'intermédiaire d'au moins une couche diélectrique (104) disposée entre le support (102) et l'élément actif (106), et entre le support (102) et le capot (114, 604) ;
- la deuxième liaison électrique comporte au moins une deuxième portion (210) de matériau électriquement conducteur reliée électriquement au capot (114, 604), disposée sur la couche diélectrique (104) et en contact électriquement avec un cordon de scellement (118) électriquement conducteur assurant une solidarisation hermétique du capot (114) au support (102).

2. Composant (100 - 600) selon la revendication 1, dans lequel l'élément actif (106) comporte au moins un circuit électronique intégré et/ou un dispositif de type MEMS/NEMS et/ou un capteur de mesure d'au moins un paramètre physiologique.

3. Composant (100 - 600) selon l'une des revendications précédentes, dans lequel le matériau du support (102) et du capot (114, 604) est du semi-conducteur dopé dont la résistivité est comprise entre environ 0,5 mOhm.cm et 20 mOhm.cm et/ou du métal.

4. Composant (100 - 600) selon l'une des revendications précédentes, dans lequel la première liaison électrique comporte au moins une première portion (110) de matériau électriquement conducteur en contact avec le support (102) et disposée au moins dans une ouverture (108) formée à travers la couche diélectrique (104).

5. Composant (100 - 600) selon l'une des revendications précédentes, dans lequel l'élément actif (106) est solidarisé au support (102) par des micro-plots (120) assurant en outre la liaison électrique entre l'élément actif (106) et le support (102) et/ou la liaison électrique entre l'élément actif (106) et le capot (114, 604), ou dans lequel l'élément actif (106) est collé sur la couche diélectrique (104) et la première et/ou la deuxième liaison électrique comporte au moins un fil électrique.

6. Composant (100 - 600) selon l'une des revendications précédentes, dans lequel le capot (114) comporte un substrat électriquement conducteur scellé hermétiquement au support (102, 202), ou dans lequel le capot (704) comporte au moins une couche mince électriquement conductrice.

7. Composant (600) selon la revendication 6, comportant en outre, lorsque le capot (604) comporte une couche mince électriquement conductrice, au moins une couche mince diélectrique (602) disposée contre la couche mince électriquement conductrice du capot (604) et entre le support (102) et la couche mince électriquement conductrice du capot (604), et dans lequel la deuxième liaison électrique traverse la couche mince diélectrique (602) via au moins une ouverture (608).

8. Composant (300) selon l'une des revendications précédentes, comportant plusieurs éléments actifs (106) encapsulés hermétiquement et individuellement dans des cavités (116) formées entre le support (102) et plusieurs capots (114) isolés électriquement les uns des autres, chacun des éléments actifs (106) étant relié électriquement à l'un des capots (114) et au support (102).

9. Composant (400) selon l'une des revendications précédentes, comportant plusieurs éléments actifs (106) encapsulés hermétiquement et individuellement dans des cavités (116) formées entre le capot (114), ou les capots (114), et plusieurs supports (102) isolés électriquement les uns des autres, chacun des éléments actifs (106) étant relié électriquement à l'un des supports (102) et au capot (206) ou à l'un des capots (206).

10. Composant (100 - 600) selon l'une des revendications précédentes, dans lequel le support (102, 202) et le capot (114, 206, 704) sont enrobés dans un matériau biocompatible tel que le composant (100 - 600) soit biocompatible et apte à être implanté dans un corps, ou au moins une partie d'un corps, humain ou animal.

11. Dispositif médical (1000) implantable dans un corps, ou au moins une partie d'un corps, humain ou animal, comportant au moins un composant (100 - 600) selon la revendication 10.

12. Procédé de réalisation d'un composant (100 - 600) comportant au moins les étapes de :
- réalisation d'au moins un élément actif (106),
- réalisation d'une première liaison électrique entre l'élément actif (106) et au moins un support (102) et d'une deuxième liaison électrique, distincte de la première liaison électrique, entre l'élément actif (106) et au moins un capot (114, 604),
- encapsulation hermétique de l'élément actif (106) dans au moins une cavité (116, 606) formée entre le support (102) et le capot (114, 604),
et dans lequel :
- le support (102) et le capot (114, 604) sont en un matériau électriquement conducteur et sont isolés électriquement l'un par rapport à l'autre ;
- l'élément actif (106) est solidarisé au support (102) par l'intermédiaire d'au moins une couche diélectrique (104) disposée entre le support (102) et l'élément actif (106), et entre le support (102) et le capot (114, 604) ;
- la deuxième liaison électrique comporte au moins une deuxième portion (210) de matériau électriquement conducteur reliée électriquement au capot (114, 604), disposée sur la couche diélectrique (104) et en contact électriquement avec un cordon de scellement (118) électriquement conducteur assurant une solidarisation hermétique du capot (114) au support (102).

13. Procédé selon la revendication 12, dans lequel le support (102) et le capot (114, 604) sont solidarisés l'un à l'autre par wafer bonding avec une activation plasma.

## Patentansprüche

1. Bauteil (100 - 600), welches mindestens ein aktives Element (106) aufweist, welches mindestens in einem Hohlraum (116, 606) hermetisch eingekapselt ist, welcher Hohlraum zwischen mindestens einem Träger (102) und mindestens einer Abdeckung (114, 604) ausgebildet ist, wobei in dem Bauteil der Träger (102) und die Abdeckung (114, 604) zumindest aus einem elektrisch leitenden Werkstoff hergestellt sind und elektrisch voneinander isoliert sind, und wobei das Bauteil eine erste elektrische Verbindung zwischen dem aktiven Element (106) und dem Träger (102) aufweist, sowie eine von der ersten elektrischen Verbindung verschiedene zweite elektrische Verbindung zwischen dem aktiven Element (106) und der Abdeckung (114, 604), und wobei in dem Bauteil:
- das aktive Element (106) mit dem Träger (102) mittels mindestens einer dielektrischen Schicht (104) verbunden ist, welche zwischen dem Träger (102) und dem aktiven Element (106) angeordnet ist, sowie zwischen dem Träger (102) und der Abdeckung (114, 604);
- die zweite elektrische Verbindung zumindest einen zweiten Abschnitt (210) aus einem elektrisch leitenden Werkstoff aufweist, welcher mit der Abdeckung (114, 604) elektrisch verbunden ist, welcher auf der dielektrischen Schicht (104) angeordnet ist und welcher in elektrischem Kontakt mit einem elektrisch leitenden Dichtungswulst (118) ist, welcher eine hermetische Verbindung der Abdeckung (114) mit dem Träger (102) sicherstellt.

2. Bauteil (100 - 600) nach Anspruch 1, in welchem das aktive Element (106) mindestens einen elektronischen integrierten Schaltkreis und/oder eine Vorrichtung vom Typ MEMS/NEMS und/oder einen Messwandler zumindest eines physiologischen Parameters aufweist.

3. Bauteil (100 - 600) nach einem der vorhergehenden Ansprüche, in welchem der Werkstoff des Trägers (102) und der Abdeckung (114, 604) ein dotierter Halbleiterwerkstoff ist, dessen spezifischer Widerstand zwischen ungefähr 0,5 mOhm.cm und 20 mOhm.cm liegt und/oder ein Metall ist.

4. Bauteil (100 - 600) nach einem der vorhergehenden Ansprüche, in welchem die erste elektrische Verbindung mindestens einen ersten Abschnitt (110) aus einem elektrisch leitenden Werkstoff in Kontakt mit dem Träger (102) aufweist, und welcher erste Abschnitt zumindest in einer Öffnung (108) angeordnet ist, welche quer durch die dielektrische Schicht (104) ausgebildet ist.

5. Bauteil (100 - 600) nach einem der vorhergehenden Ansprüche, in welchem das aktive Element (106) mit dem Träger (102) über Mikrostifte (120) verbunden ist, welche zudem die elektrische Verbindung zwischen dem aktiven Element (106) und der Abdeckung (114, 604) sicherstellen, und in welchem das aktive Element (106) mit der dielektrischen Schicht (104) verklebt ist und die erste und/oder die zweite elektrische Verbindung mindestens einen elektrischen Draht aufweist.

6. Bauteil (100 - 600) nach einem der vorhergehenden Ansprüche, in welchem die Abdeckung (114) ein elektrisch leitendes Substrat aufweist, welches hermetisch gegen den Träger (102, 202) abgedichtet ist, oder in welchem die Abdeckung (704) mindestens eine elektrisch leitende Dünnschicht aufweist.

7. Bauteil (600) nach Anspruch 6, welches des Weiteren, wenn die Abdeckung (604) eine elektrisch leitende Dünnschicht aufweist, mindestens eine dielektrische Dünnschicht (602) aufweist, welche gegen die elektrisch leitende Dünnschicht der Abdeckung (604) und zwischen dem Träger (102) und der elektrisch leitenden Dünnschicht der Abdeckung (604) angeordnet ist, und in welchem die zweite elektrische Verbindung die dielektrische Dünnschicht (602) über mindestens eine Öffnung (608) durchdringt.

8. Bauteil (300) nach einem der vorhergehenden Ansprüche, welches mehrere aktive Elemente (106) aufweist, welche in den Hohlräumen (116) hermetisch und einzeln eingekapselt sind, wobei die Hohlräume zwischen dem Träger (102) und mehreren voneinander elektrisch isolierten Abdeckungen (114) ausgebildet sind, wobei jedes der aktiven Elemente (106) mit einer der Abdeckungen (114) und mit dem Träger (102) elektrisch verbunden ist.

9. Bauteil (400) nach einem der vorhergehenden Ansprüche, welches mehrere aktive Elemente (106) aufweist, welche hermetisch und einzeln in den Hohlräumen (116) eingekapselt sind, wobei die Hohlräume zwischen der Abdeckung (114), oder den Abdeckungen (114), und mehreren Trägern (102) ausgebildet sind, welche voneinander elektrisch isoliert sind, wobei jedes der aktiven Elemente (106) mit einem der Träger (102) und mit der Abdeckung (206) oder mit einer der Abdeckungen (206) elektrisch verbunden ist.

10. Bauteil (100 - 600) nach einem der vorhergehenden Ansprüche, in welchem der Träger (102, 202) und die Abdeckung (114, 206, 704) in einem biokompatiblen Werkstoff verpackt sind, so dass das Bauteil (100 - 600) biokompatibel und geeignet ist, um in einen Körper, oder zumindest einen Teil eines Körpers, menschlich oder animalisch, implantiert zu werden.

11. Medizinische Vorrichtung (1000), welche in einen Körper, oder zumindest in einen Teil eines Körpers, menschlich oder animalisch, implantierbar ist, wobei die Vorrichtung mindestens ein Bauteil (100 - 600) nach Anspruch 10 aufweist.

12. Verfahren zur Herstellung eins Bauteils (100 - 600), welches zumindest die folgenden Schritte umfasst:
- Herstellung mindestens eines aktiven Elements (106),
- Herstellung einer ersten elektrischen Verbindung zwischen dem aktiven Element (106) und zumindest einem Träger (102) und einer zweiten elektrischen Verbindung, welche von der ersten elektrischen Verbindung verschieden ist, zwischen dem aktiven Element (106) und mindestens einer Abdeckung (114, 604);
- Hermetische Einkapselung des aktiven Elements (106) in mindestens einem Hohlraum (116, 606), welcher zwischen dem Träger (102) und der Abdeckung (114, 604) ausgebildet ist;
und in welchem:
- der Träger (102) und die Abdeckung (114, 604) aus einem elektrisch leitenden Werkstoff sind und voneinander elektrisch isoliert sind;
- das aktive Element (106) mit dem Träger (102) mittels mindestens einer dielektrischen Schicht (104) verbunden ist, welche zwischen dem Träger (102) und dem aktiven Element (106) angeordnet ist, und zwischen dem Träger (102) und der Abdeckung (114, 604);
- die zweite elektrische Verbindung mindestens einen zweiten Abschnitt (210) aus einem elektrisch leitenden Werkstoff aufweist, welcher mit der Abdeckung (114, 604) elektrisch verbunden ist, welcher auf der dielektrischen Schicht (104) angeordnet ist und in elektrischem Kontakt mit einer elektrisch leitenden Dichtungswulst (118) ist, welche eine hermetische Verbindung der Abdeckung (114) mit dem Träger (102) sicherstellt.

13. Verfahren nach Anspruch 12, in welchem der Träger (102) und die Abdeckung (114, 604) durch Wafer Bonding mit Plasmaaktivierung miteinander verbunden werden.

## Claims

1. Component (100 - 600) including at least one active element (106) hermetically encapsulated in at least one cavity (116, 606) formed between at least one support (102) and at least one cover (114, 604), in which the support (102) and the cover (114, 604) are made from at least one electrically conductive material, and are insulated electrically from one another, and include a first electrical connection between the active element (106) and the support (102), and a second electrical connection, separate from the first electrical connection, between the active element (106) and the cover (114, 604), and in which:
- the active element (106) is securely attached to the support (102) through at least one dielectric layer (104) positioned between the support (102) and the active element (106), and between the support (102) and the cover (114, 604);
- the second electrical connection includes at least one second portion (210) of electrically conductive material electrically connected to the cover (114, 604), positioned on the dielectric layer (104) and electrically in contact with an electrically conductive sealing bead (118) providing hermetic secure attachment of the cover (114) to the support (102).

2. Component (100 - 600) according to claim 1, in which the active element (106) includes at least an integrated electronic circuit and/or a device of the MEMS/NEMS type and/or a sensor measuring at least one physiological parameter.

3. Component (100 - 600) according to one of previous claims, in which the material of the support (102) and of the cover (114, 604) is a doped semiconductor, the resistivity of which is between approximately 0.5 mOhm.cm and 20 mOhm.cm, and/or a metal.

4. Component (100 - 600) according to one of previous claims, in which the first electrical connection includes at least a first portion (110) of electrically conductive material in contact with the support (102) and positioned at least in one aperture (108) formed through the dielectric layer (104).

5. Component (100 - 600) according to one of previous claims, in which the active element (106) is attached to the support (102) by micro-terminals (120) also providing the electrical connection between the active element (106) and the support (102) and/or the electrical connection between the active element (106) and the cover (114, 604), or in which the active element (106) is bonded on the dielectric layer (104) and the first and/or the second electrical connection includes at least one electric wire.

6. Component (100 - 600) according to claim 1, in which the cover (114) includes an electrically conductive substrate attached hermetically to the support (102, 202), or in which the cover (704) includes at least one electrically conductive thin layer.

7. Component (600) according to claim 6, also including, when the cover (604) includes an electrically conductive thin layer, at least one dielectric thin layer (602) positioned against the electrically conductive thin layer of the cover (604) and between the support (102) and the electrically conductive thin layer of the cover (604), and in which the second electrical connection comes through the dielectric thin layer (602) via at least one aperture (608).

8. Component (300) according to one of previous claims, including several active elements (106) which are encapsulated hermetically and individually in cavities (116) formed between the support (102), and several covers (114) which are electrically insulated from one another, where each of the active elements (106) is connected electrically to one of the covers (114) and to the support (102).

9. Component (400) according to one of previous claims, including several active elements (106) which are encapsulated hermetically and individually in cavities (116) formed between the cover (114), or the covers (114), and several supports (102) which are electrically insulated from one another, where each of the active elements (106) is connected electrically to one of the supports (102) and to the cover (206) or to one of the covers (206).

10. Component according to one of previous claims, in which the support (102, 202) and the cover (114, 206, 704) are coated in a biocompatible material such that the component (100 - 600) is biocompatible and able to be implanted in a body, or at least a part of a body, whether human or animal.

11. Medical device (1000) which is implantable in a body, or at least a part of a body, whether human or animal, including at least one component (100 - 600) according to claim 10.

12. Method for producing a component (100 - 600), including at least the following steps:
- production of at least one active element (106),
- production of a first electrical connection between the active element (106) and at least one support (102) and of a second electrical connection, separate from the first electrical connection, between the active element (106) and at least one cover (114, 604),
- hermetic encapsulation of the active element (106) in at least one cavity (116, 606) formed between the support (102) and the cover (114, 604),
and in which:
- the support (102) and the cover (114, 604) are made from an electrically conductive material and are electrically insulated from one another;
- the active element (106) is securely attached to the support (102) through at least one dielectric layer (104) positioned between the support (102) and the active element (106) and between the support (102) and the cover (114, 604);
- the second electrical connection includes at least one second portion (210) of electrically conductive material electrically connected to the cover (114, 604), positioned on the dielectric layer (104) and electrically in contact with an electrically conductive sealing bead (118) providing hermetic secure attachment of the cover (114) to the support (102).

13. Method according to claim 12, in which the support (102) and the cover (114, 604) are securely attached to one another by wafer bonding with plasma activation.
